# EUROPEAN PATENT APPLICATION

(11) **EP 4 706 601 A1**
(43) Date of publication of application: **11.03.2026**
(21) Application number: 24800281.8
(22) Date of filing: 21.05.2024
(51) Int. Cl.: A61F 2/38, A61F 2/48, A61F 2/30

(54) **BIO-IMPLANTABLE ACTUATOR AND ARTIFICIAL JOINT COMPRISING SAME**

(30) Priority: 04.05.2023 KR 20230058789; 04.05.2023 KR 20230058790
(71) Applicant: Kyungpook National University Industry-Academic Cooperation Foundation, Daegu 41566 (KR)
(72) Inventor: LEE, Hyun Joo, Daegu 42116 (KR); LEE, Hyun Woo, Daegu 41185 (KR); PARK, Chul Woo, Daegu 42195 (KR); KYUNG, Hee Soo, Gunwi-gun Gyeongsangbuk-do 39034 (KR)
(74) Representative: Regimbeau
(86) International application number: PCT/KR2024/006874
(87) International publication number: WO 2024/228600

(57) **Abstract**

The present invention relates to a bio-implantable actuator and an artificial joint including the same, and more particularly, to a bio-implantable actuator, which is capable of transmitting a movement force and reducing a load applied to a joint without limiting the degree of freedom of movement of the joint, and an artificial joint including the same. A bio-implantable actuator according to one embodiment of the present invention includes a power section generating a rotational force, a linear movement section that is connected to the power section and converts rotational movement of the power section into linear movement, and a flexible rod connected to the linear movement section. In addition, a bio-implantable actuator according to another embodiment of the present invention includes a tube having one open end and a hollow portion, a linear movement section disposed inside the tube, and a flexible rod having one end disposed inside the tube and connected to the linear movement section and another end protruding to the outside of the tube.

## Description

### [Technical Field]

The present invention relates to a bio-implantable actuator and an artificial joint including the same, and more particularly, to a bio-implantable actuator, which is capable of transmitting a movement force and reducing a load applied to a joint while not limiting the degree of freedom of movement of the joint, and an artificial joint including the same.

### [Background Art]

Artificial knee joint replacement surgery is a surgical procedure for replacing a knee joint that is damaged due to trauma or a disease, such as degenerative arthritis, osteonecrosis, or a meniscal tear, with an artificial joint.

At this time, the artificial joint consists of a femur coupling member coupled to a femur, a tibia coupling member coupled to a tibia, and a bearing member that serves as cartilage between the femur coupling member and the tibia coupling member.

Meanwhile, a patient with a movement disorder due to muscle loss caused by trauma, a disease, or aging, in addition to damage to a knee joint, has difficulty walking even after receiving artificial knee joint replacement surgery, and there is a problem that hyperextension of the knee may occur, which can lead to genu recurvatum.

### [Disclosure]

### [Technical Problem]

The present invention is for addressing the above problems and is directed to providing a bio-implantable actuator, which is capable of transmitting a movement force and reducing a load applied to a joint without limiting the degree of freedom of movement of the joint, and an artificial joint including the same.

### [Technical Solution]

According to one aspect of the present invention for achieving the above objective, there is provided a bio-implantable actuator that is coupled to and implanted in an artificial joint, the bio-implantable actuator including a tube having one open end and a hollow portion, a power section that is disposed inside the tube and generates a rotational force, a linear movement section that is disposed inside the tube, is connected to the power section, and converts rotational movement of the power section into linear movement, and a flexible rod having one end that is disposed inside the tube and comes into contact with the linear movement section and another end that protrudes to the outside of the tube, wherein the linear movement section is provided to press the flexible rod during rotation of the power section in a first direction.

In addition, according to one aspect of the present invention, the linear movement section may release a pressure applied to the flexible rod during rotation of the power section in a second direction that is a direction opposite to the first direction.

In addition, according to one aspect of the present invention, the linear movement section may include a screw rotatably connected to the power section, a moving member connected to move linearly according to rotation of the screw, a first connecting member connected to the one end of the flexible rod, and an elastic member coupled between the moving member and the first connecting member.

In addition, according to one aspect of the present invention, the elastic member may be compressed or stretched according to movement of the moving member and may compress or stretch the flexible rod by an elastic force.

In addition, in the bio-implantable actuator according to one aspect of the present invention, during the rotation of the power section in the first direction, the moving member may move toward the flexible rod, and the elastic member may move the first connecting member to the flexible rod.

In addition, in the bio-implantable actuator according to one aspect of the present invention, during the rotation of the power section in the second direction, the moving member may move toward the power section.

In addition, according to one aspect of the present invention, the tube may include a coupling hole for fixing to a femur.

In addition, according to one aspect of the present invention, the power section may include a motor transmitting a rotational force to the screw and a motor fixing member fixing the motor to the tube.

In addition, according to another aspect of the present invention, there is provided a bio-implantable actuator including a tube having one open end and a hollow portion, a linear movement section disposed inside the tube, and a flexible rod having one end that is disposed inside the tube and connected to the linear movement section and another end that protrudes to the outside of the tube, wherein the linear movement section includes an elastic member provided to be compressed when the flexible rod is bent.

In addition, the bio-implantable actuator according to another aspect of the present invention may include a tension adjuster that is able to adjust tension of the elastic member.

In addition, according to another aspect of the present invention, the tube may include a coupling hole for fixing to a femur and an adjustment hole connecting the hollow portion of the tube and an outer portion of the tube.

In addition, according to another aspect of the present invention, the tension adjuster may include a tension adjustment bolt disposed in the adjustment hole of the tube, a screw disposed inside the tube, and a second connecting member that is formed of a soft material and connects the tension adjustment bolt and the screw.

In addition, according to another aspect of the present invention, the linear movement section may further include a third connecting member that is movably disposed at the screw and is coupled to the flexible rod.

In addition, according to another aspect of the present invention, the tension adjuster may further include an adjustment nut adjusting a compressed length of the elastic member and a pin coupling the adjustment nut to the third connecting member, and the third connecting member may include a pin hole to which the pin is coupled.

Meanwhile, according to one aspect of the present invention for achieving the above objective, there is provided an artificial joint including a bio-implantable actuator, a femur connector fixed to a lower end of a femur, a tibia tray fixed to an upper end of a tibia, and a bearing that is coupled to an upper end of the tibia tray and has a curved surface formed thereon, wherein the bio-implantable actuator includes a tube having one open end and a hollow portion, a power section that is disposed inside the tube and generates a rotational force, a linear movement section that is disposed inside the tube, is connected to the power section, and converts rotational movement of the power section into linear movement, and a flexible rod having one end that is disposed inside the tube and comes into contact with the linear movement section and the other end that protrudes to the outside of the tube, the bio-implantable actuator is coupled to the tibia tray, and the femur connector is disposed to be slidable along the curved surface of the bearing.

In addition, according to one aspect of the present invention, the bearing may have a through-hole through which the tube and the flexible rod pass.

In addition, according to one aspect of the present invention, a width of the through-hole may be larger than a diameter of the tube.

In addition, according to one aspect of the present invention, the tibia tray may include a first coupling groove coupled to the through-hole of the bearing and a second coupling groove coupled to the flexible rod.

In addition, according to one aspect of the present invention, the second coupling groove may have a narrowing width on at least a portion thereof in a direction toward a bottom surface and may guide a bending direction of the flexible rod.

In addition, according to one aspect of the present invention, the flexible rod may be bent along the second coupling groove upon receiving a pressure.

In addition, according to one aspect of the present invention, the flexible rod may convert linear movement of the linear movement section into rotational movement.

In addition, in the artificial joint according to one aspect of the present invention, during rotation of the power section in a first direction, the linear movement section may press the flexible rod, and the pressed flexible rod may be bent.

In addition, in the artificial joint according to one aspect of the present invention, during rotation of the power section in a second direction that is a direction opposite to the first direction, the linear movement section may release the pressure applied to the flexible rod, and the flexible rod from which the applied pressure is released may be unbent.

In addition, according to another aspect of the present invention, there is provided an artificial joint including a bio-implantable actuator, a femur connector fixed to a lower end of a femur, a tibia tray fixed to an upper end of a tibia, and a bearing that is coupled to an upper end of the tibia tray and has a curved surface formed thereon, wherein the bio-implantable actuator includes a tube having a hollow portion, a linear movement section disposed inside the tube, and a flexible rod having one end that is disposed inside the tube and comes into contact with the linear movement section and the other end that protrudes to the outside of the tube, the bio-implantable actuator is coupled to the tibia tray, the linear movement section includes an elastic member provided to be compressed when the flexible rod is bent, and the femur connector is disposed to be slidable along the curved surface of the bearing.

In addition, in the artificial joint according to another aspect of the present invention, during a bending operation of the artificial joint, the flexible rod may be bent, and the bent flexible rod may compress the elastic member.

Further, in the artificial joint according to another aspect of the present invention, during an unbending operation of the artificial joint, the flexible rod may be unbent, and the unbent flexible rod may loosen the elastic member.

### [Advantageous Effects]

A bio-implantable actuator according to the present invention can transmit a movement force without limiting the degree of freedom of movement of a joint.

In addition, a bio-implantable actuator according to the present invention can reduce a load applied to a joint.

### [Description of Drawings]

FIG. 1 is a view showing a state in which an artificial joint according to one embodiment of the present invention is coupled to a femur and a tibia.
FIG. 2 is an exploded perspective view of the artificial joint according to one embodiment of the present invention.
FIG. 3 is a view showing a cross-section of a tibia tray according to one embodiment of the present invention.
FIG. 4 is a perspective view showing a state in which a bio-implantable actuator and the tibia tray are coupled to each other according to one embodiment of the present invention.
FIG. 5 is a view showing the bio-implantable actuator according to one embodiment of the present invention.
FIG. 6 is an exploded perspective view of the bio-implantable actuator according to one embodiment of the present invention.
FIG. 7 is a cross-sectional view of the bio-implantable actuator according to one embodiment of the present invention.
FIGS. 8 and 9 are views showing operation states of the artificial joint according to one embodiment of the present invention.
FIG. 10 is a view showing a bio-implantable actuator according to another embodiment of the present invention.
FIG. 11 is an exploded perspective view of the bio-implantable actuator according to another embodiment of the present invention.
FIG. 12 is a cross-sectional view of the bio-implantable actuator according to another embodiment of the present invention.
FIG. 13 is a cross-sectional view of a third connecting member along line A-A' of FIG. 11.

### [Modes of the Invention]

Hereinafter, exemplary embodiments of the present invention will be described in more detail with reference to the accompanying drawings.

In addition, the same or corresponding components are given the same or similar reference numerals throughout the drawings, and redundant descriptions thereof are omitted, and for convenience of description, the size and shape of each component illustrated in the drawings may be exaggerated or reduced.

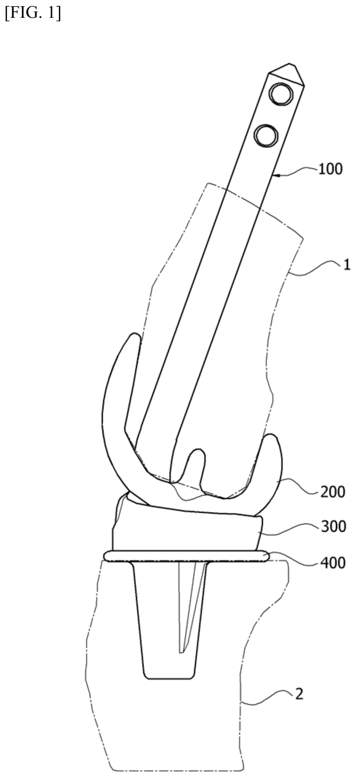

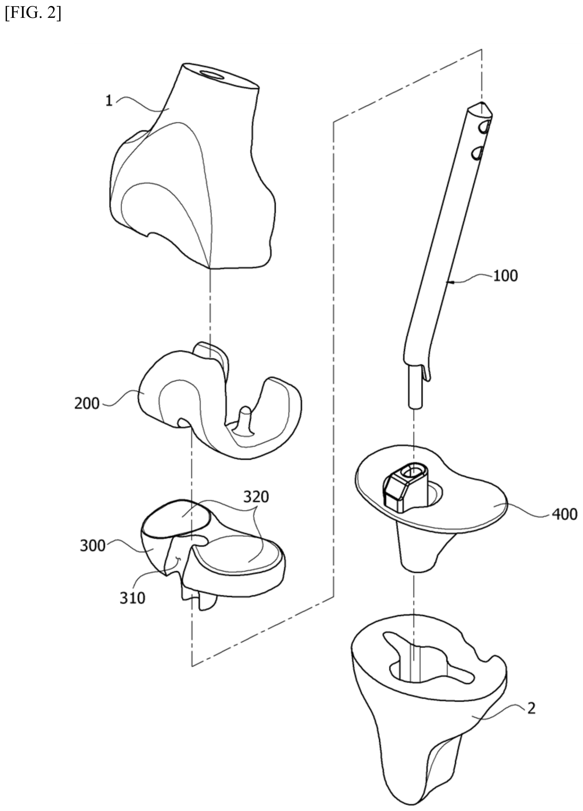

FIG. 1 is a view showing a state in which an artificial joint according to one embodiment of the present invention is coupled to a femur and a tibia, and FIG. 2 is an exploded perspective view of the artificial joint according to one embodiment of the present invention.

Referring to FIGS. 1 and 2, the artificial joint according to one embodiment of the present invention may include a bio-implantable actuator 100, a femur connector 200 fixed to a lower end of a femur 1, a tibia tray 400 fixed to an upper end of a tibia 2, and a bearing 300 that is coupled to an upper end of the tibia tray 400 and has a curved surface 320 formed thereon.

At this time, the bio-implantable actuator 100 may be coupled to the tibia tray 400.

The femur connector 200 according to one embodiment of the present invention may be disposed to be slidable along the curved surface 320 of the bearing 300.

The bearing 300 according to one embodiment of the present invention may have a through-hole 310 through which a tube 110 and a flexible rod 140 pass.

At this time, a width of the through-hole 310 may be larger than a diameter of the tube 110.

In addition, the through-hole 310 may have a form that is open toward the front of a knee and extends downward.

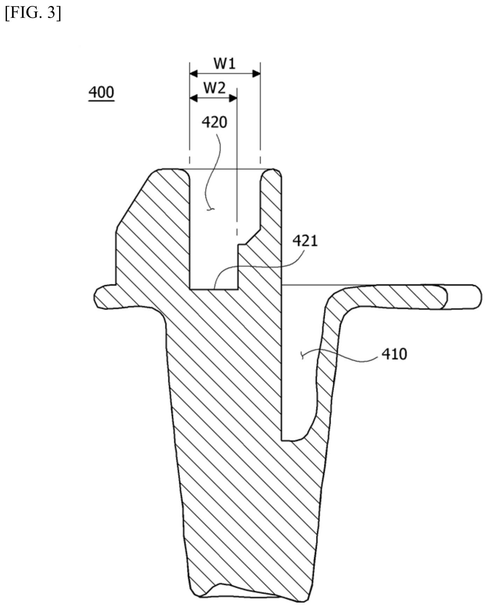

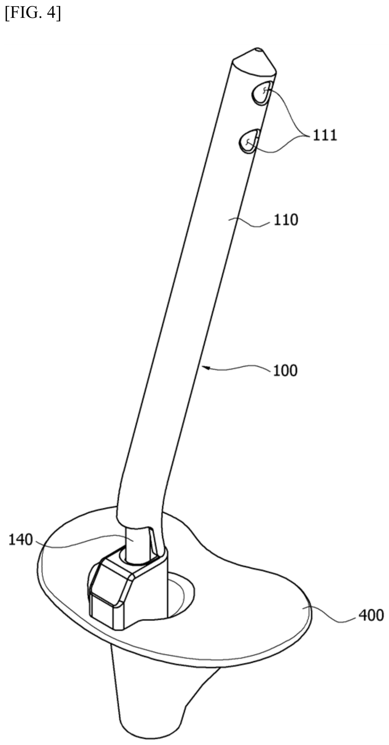

FIG. 3 is a view showing a cross-section of a tibia tray according to one embodiment of the present invention, and FIG. 4 is a perspective view showing a state in which a bio-implantable actuator and the tibia tray are coupled to each other according to one embodiment of the present invention.

Referring to FIGS. 1 to 4, the tibia tray 400 according to one embodiment of the present invention may include a first coupling groove 410 coupled to the through-hole 310 of the bearing 300 and a second coupling groove 420 coupled to the flexible rod 140 which will be described below.

Specifically, an extending portion of the through-hole 310 of the bearing 300 may be coupled to the first coupling groove 410 of the tibia tray 400.

In addition, the second coupling groove 420 of the tibia tray 400 may have a width that changes in a direction toward a bottom surface 421.

Specifically, the second coupling groove 420 may have a narrowing width on at least a portion thereof in the direction toward the bottom surface 421. Accordingly, a width W1 of an open surface of the second coupling groove 420 may be larger than a width W2 of the bottom surface 421.

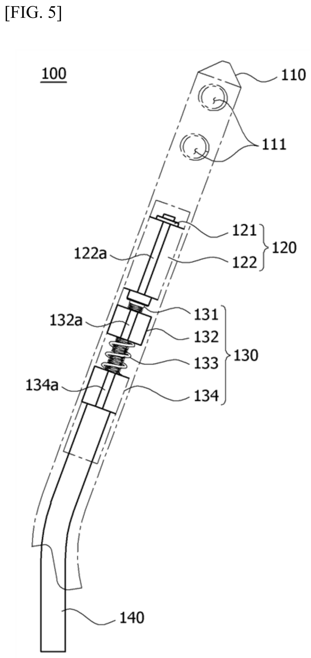

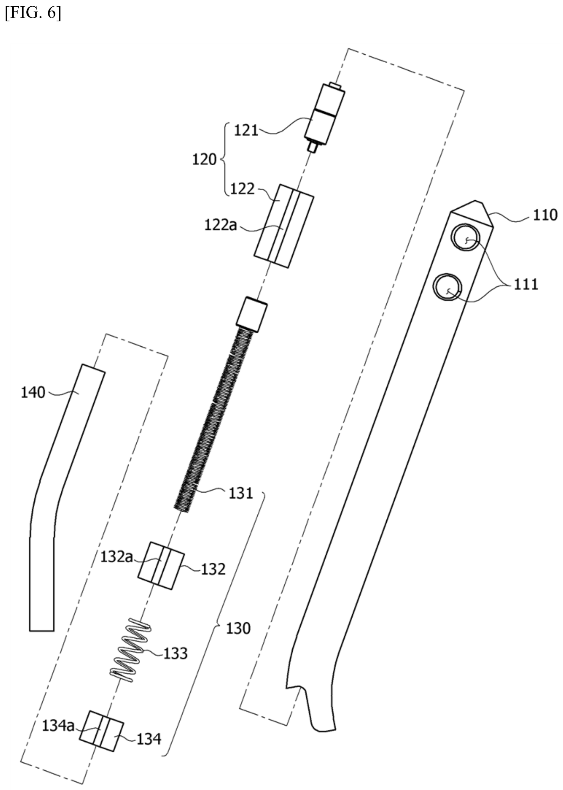

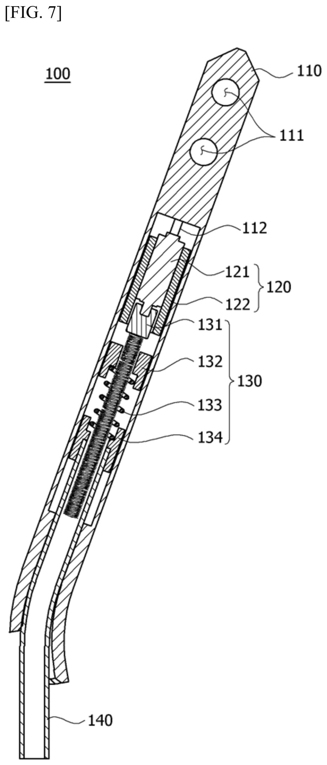

FIG. 5 is a view showing the bio-implantable actuator according to one embodiment of the present invention, FIG. 6 is an exploded perspective view of the bio-implantable actuator according to one embodiment of the present invention, and FIG. 7 is a cross-sectional view of the bio-implantable actuator according to one embodiment of the present invention.

Referring to FIGS. 5 to 7, the bio-implantable actuator 100 according to one embodiment of the present invention may include the tube 110 having one open end and a hollow portion, a power section 120 that is disposed inside the tube 110 and generates a rotational force, a linear movement section 130 that is disposed inside the tube 110, is connected to the power section 120, and converts rotational movement of the power section 120 into linear movement, and a flexible rod 140 having one end that is disposed inside the tube 110 and comes into contact with the linear movement section 130 and another end that protrudes to the outside of the tube 110.

The tube 110 may include a coupling hole 111 for fixing to the femur 1.

At this time, the tube 110 may be fixed to the femur 1 through a fastening member screw-coupled to the coupling hole 111.

In addition, the tube 110 may have one or more guide protrusions 112 formed on an inner circumferential surface thereof.

The power section 120 may include a motor 121 transmitting a rotational force to a screw 131, which will be described below, and a motor fixing member 122 fixing the motor 121 to the tube 110.

At this time, the motor fixing member 122 may be coupled to an inner portion of the tube 110, and specifically, the motor fixing member 122 may have one or more guide grooves 122a formed in an outer circumferential surface thereof. The guide grooves 122a may accommodate the guide protrusions 112 of the tube 110. As a result, the motor fixing member 122 can be firmly coupled to the tube 110.

In addition, the motor 121 may be fixed by being fitted and coupled to the motor fixing member 122.

The linear movement section 130 may include the screw 131 rotatably connected to the power section 120, a moving member 132 connected to move linearly according to rotation of the screw 131, a first connecting member 134 connected to the one end of the flexible rod 140, and an elastic member 133 coupled between the moving member 132 and the first connecting member 134.

At this time, by the linear movement section 130 including the elastic member 133, the bio-implantable actuator 100 can transmit a movement force without limiting the degree of freedom of movement of a joint.

In addition, the linear movement section 130 may convert rotational movement of the power section 120 into linear movement.

Specifically, the screw 131 may be connected to the motor 121 of the power section 120 and may rotate together as the motor rotates.

In addition, the moving member 132 may be connected to move linearly in the longitudinal direction of the screw 131 as the screw 131 rotates.

At this time, the tube 110 may guide the moving member 132 to move linearly.

In addition, the moving member 132 may have one or more guide grooves 132a formed in an outer circumferential surface thereof. The guide grooves 132a may accommodate the guide protrusions 112 of the tube 110. As a result, rotational movement of the moving member 132 can be limited by the tube 110, and the moving member 132 can be induced to be capable of linear movement only.

Meanwhile, the first connecting member 134 connected to the one end of the flexible rod 140 may be disposed inside the tube 110 and may be coupled to the tube 110.

At this time, the first connecting member 134 may have one or more guide grooves 134a formed in an outer circumferential surface thereof. The guide grooves 134a may accommodate the guide protrusions 112 of the tube 110. As a result, the first connecting member 134 can be firmly coupled to the tube 110.

In addition, the elastic member 133 may be compressed or stretched according to movement of the moving member 132 and may compress or stretch the flexible rod 140 by an elastic force.

The flexible rod 140 may convert linear movement of the linear movement section 130 into rotational movement.

Specifically, the flexible rod 140 may be bent by receiving a pressure from the first connecting member 134.

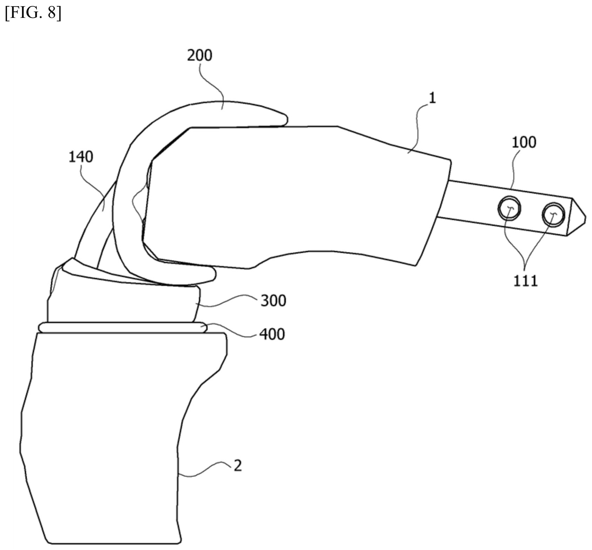

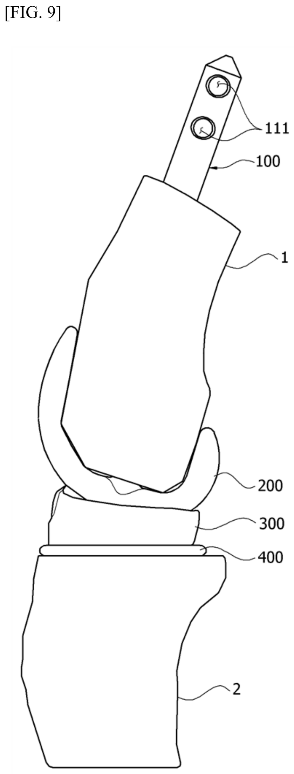

FIGS. 8 and 9 are views showing operation states of the artificial joint according to one embodiment of the present invention.

Next, an operation process of the artificial joint according to one embodiment of the present invention will be described with reference to FIGS. 8 and 9.

In the bio-implantable actuator 100, during rotation of the power section 120 in a first direction, the linear movement section 130 may press the flexible rod 140, and the pressed flexible rod 140 may be bent.

Specifically, during the rotation of the power section 120 in the first direction, the moving member 132 may move toward the flexible rod 140, and the elastic member 133 may move the first connecting member 134 to the flexible rod 140.

At this time, the elastic member 133 compressed by the moving member 132 may compress the flexible rod 140 by an elastic force, and the compressed flexible rod 140 may be bent.

In addition, the second coupling groove 420 of the tibia tray 400 may guide a bending direction of the flexible rod 140.

At this time, a diameter of the flexible rod 140 coupled to the second coupling groove 420 may be equal to the width W2 of the bottom surface 421 of the second coupling groove 420, and the flexible rod 140 that has received pressure may be guided toward an empty space of the second coupling groove 420 and may be bent.

Due to being bent as described above, the flexible rod 140 can convert linear movement of the linear movement section 130 into rotational movement.

In addition, due to the flexible rod 140 being bent, a user of the artificial joint can be compensated with a movement force necessary for bending a knee joint.

Meanwhile, in the bio-implantable actuator 100, during rotation of the power section 120 in a second direction that is a direction opposite to the first direction, the linear movement section 130 may release the pressure applied to the flexible rod 140, and the flexible rod 140 from which the applied pressure is released may be unbent.

Specifically, during the rotation of the power section 120 in the second direction, the moving member 132 may move toward the power section 120.

At this time, the elastic member 133 from which the applied pressure is released may release the pressure applied to the flexible rod 140, and the flexible rod 140 from which the applied pressure is released may be unbent.

In addition, due to the flexible rod 140 being unbent, the user of the artificial joint can be compensated with a movement force necessary for unbending the knee joint.

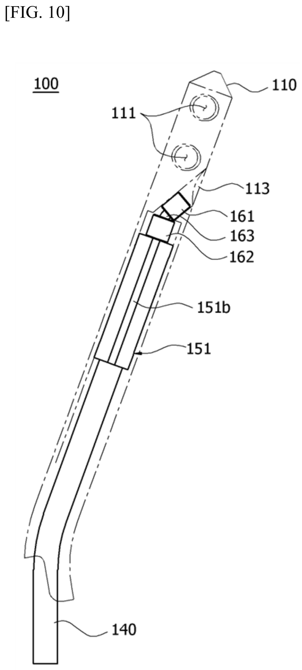

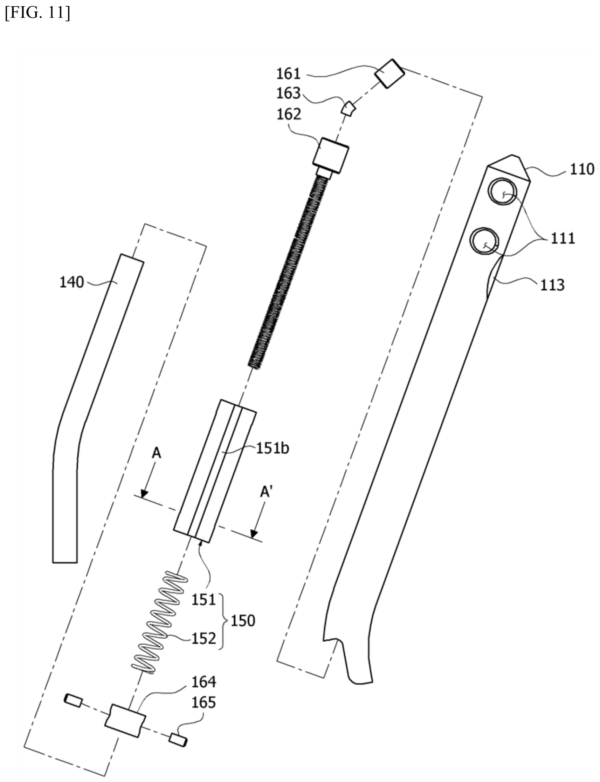

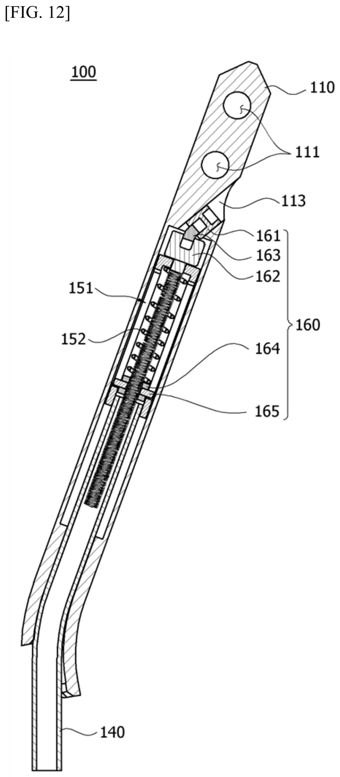

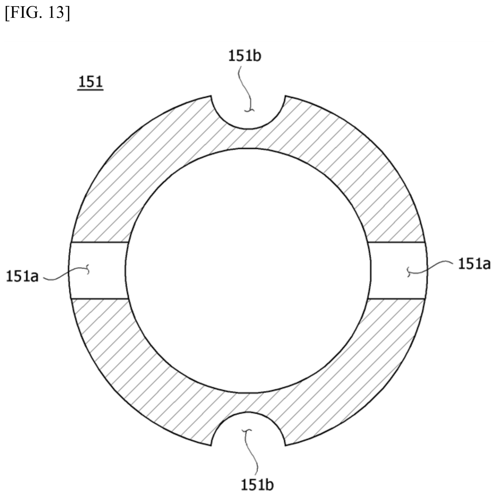

FIG. 10 is a view showing a bio-implantable actuator according to another embodiment of the present invention, FIG. 11 is an exploded perspective view of the bio-implantable actuator according to another embodiment of the present invention, FIG. 12 is a cross-sectional view of the bio-implantable actuator according to another embodiment of the present invention, and FIG. 13 is a cross-sectional view of a third connecting member along line A-A' of FIG. 11.

Next, a bio-implantable actuator and an artificial joint according to another embodiment of the present invention will be described with reference to FIGS. 8 to 13, redundant description will be omitted, and description will be given focusing on differences from the bio-implantable actuator and the artificial joint according to one embodiment of the present invention that have been described above.

A bio-implantable actuator 100 according to another embodiment of the present invention may include a tube 110 having one open end and a hollow portion, a linear movement section 150 disposed inside the tube 110, and a flexible rod 140 having one end that is disposed inside the tube 110 and connected to the linear movement section 150 and another end that protrudes to the outside of the tube 110.

At this time, the linear movement section 150 may include an elastic member 152 provided to be compressed when the flexible rod 140 is bent. The elastic member 152 may be a spring that serves as a counterbalance.

At this time, by the linear movement section 150 including the elastic member 152, the bio-implantable actuator 100 can transmit a movement force without limiting the degree of freedom of movement of a joint.

Meanwhile, the linear movement section 150 may further include a third connecting member 151 that is movably disposed at a screw 162, which will be described below, and is coupled to the flexible rod 140.

At this time, the third connecting member 151 may be in the form of a tube having both sides open and a hollow portion.

In addition, the third connecting member 151 may include a pin hole 151a to which a pin 165, which will be described below, is coupled.

In addition, the third connecting member 151 may have one or more guide grooves 151b formed in an outer circumferential surface thereof. The guide grooves 151b may accommodate guide protrusions of the tube 110. As a result, rotational movement of the third connecting member 151 can be limited by the tube 110, and the third connecting member 151 can be induced to be capable of linear movement only.

In addition, the bio-implantable actuator 100 according to another embodiment of the present invention may include a tension adjuster 160 that is able to adjust tension of the elastic member 152.

Meanwhile, the tube 110 may include a coupling hole 111 for fixing to a femur 1.

At this time, the tube 110 may be fixed to the femur 1 through a fastening member screw-coupled to the coupling hole 111.

In addition, the tube 110 may have one or more guide protrusions formed on an inner circumferential surface thereof.

In addition, the tube 110 may include an adjustment hole 112 connecting the hollow portion of the tube 110 and an outer portion of the tube 110.

The adjustment hole 112 may be formed to pass through an upper surface of the hollow portion of the tube 110 and a sidewall of the other end of the tube 110.

Meanwhile, the tension adjuster 160 may include a tension adjustment bolt 161 disposed in the adjustment hole 112 of the tube 110, the screw 162 disposed inside the tube 110, a second connecting member 163 that is formed of a soft material and connects the tension adjustment bolt 161 and the screw 162, an adjustment nut 164 adjusting a compressed length of the elastic member 152, and the pin 165 coupling the adjustment nut 164 to the third connecting member 151.

Meanwhile, in the bio-implantable actuator 100 according to another embodiment of the present invention, an initial position of the adjustment nut 164 may be determined by manipulating the tension adjustment bolt 161 through the adjustment hole 112. In this way, tension of the elastic member 152 may be determined by adjusting the compressed length of the elastic member 152.

In the artificial joint according to another embodiment of the present invention, during a bending operation of the artificial joint, the flexible rod 140 of the bio-implantable actuator 100 may be bent, and as the flexible rod 140 is bent, the third connecting member 151 coupled to the flexible rod 140 may move in a direction in which the tube 110 opens.

At this time, the elastic member 152 may be compressed between the third connecting member 151 and the adjustment nut 164 that is fixed.

The elastic member 152 compressed in this way may store energy in the form of an elastic force.

Meanwhile, during an unbending operation of the artificial joint, the flexible rod 140 of the bio-implantable actuator 100 may be unbent, and as the flexible rod 140 is unbent, the third connecting member 151 coupled to the flexible rod 140 may move in a direction opposite to the direction in which the tube 110 opens.

At this time, the elastic member 152 may be loosened between the third connecting member 151 and the adjustment nut 164 that is fixed.

The elastic member 152 loosened in this way may release the energy stored in the form of an elastic force.

By the method described above, the bio-implantable actuator 100 can reduce a load applied to a joint during a bending operation and an unbending operation of the artificial joint.

The exemplary embodiments of the present invention that have been described above are disclosed only for illustrative purposes, various modifications and changes within the technical spirit of the present invention may be made by those of ordinary skill in the art to which the present invention pertains, and such modifications and changes fall within the scope of the present invention.

### [Industrial Applicability]

A bio-implantable actuator related to an embodiment of the present invention can transmit a movement force and reduce a load applied to a joint without limiting the degree of freedom of movement of the joint. The bio-implantable actuator can be applied to an artificial joint used in artificial knee joint replacement surgery for replacing a damaged knee joint.

## Claims

1. A bio-implantable actuator that is coupled to and implanted in an artificial joint, the bio-implantable actuator comprising:
a tube having one open end and a hollow portion;
a power section that is disposed inside the tube and generates a rotational force;
a linear movement section that is disposed inside the tube, is connected to the power section, and converts rotational movement of the power section into linear movement; and
a flexible rod having one end that is disposed inside the tube and comes into contact with the linear movement section and another end that protrudes to the outside of the tube,
wherein the linear movement section is provided to press the flexible rod during rotation of the power section in a first direction.

2. The bio-implantable actuator of claim 1, wherein the linear movement section releases a pressure applied to the flexible rod during rotation of the power section in a second direction that is a direction opposite to the first direction.

3. The bio-implantable actuator of claim 2, wherein the linear movement section includes:
a screw rotatably connected to the power section;
a moving member connected to move linearly according to rotation of the screw;
a first connecting member connected to the one end of the flexible rod; and
an elastic member coupled between the moving member and the first connecting member.

4. The bio-implantable actuator of claim 3, wherein the elastic member is compressed or stretched according to movement of the moving member and compresses or stretches the flexible rod by an elastic force.

5. The bio-implantable actuator of claim 4, wherein, during the rotation of the power section in the first direction, the moving member moves toward the flexible rod, and the elastic member moves the first connecting member to the flexible rod.

6. The bio-implantable actuator of claim 4, wherein, during the rotation of the power section in the second direction, the moving member moves toward the power section.

7. The bio-implantable actuator of claim 1, wherein the tube includes a coupling hole for fixing to a femur.

8. The bio-implantable actuator of claim 3, wherein the power section includes:
a motor transmitting a rotational force to the screw; and
a motor fixing member fixing the motor to the tube.

9. A bio-implantable actuator that is coupled to and implanted in an artificial joint, the bio-implantable actuator comprising:
a tube having one open end and a hollow portion;
a linear movement section disposed inside the tube; and
a flexible rod having one end that is disposed inside the tube and connected to the linear movement section and another end that protrudes to the outside of the tube,
wherein the linear movement section includes an elastic member provided to be compressed when the flexible rod is bent.

10. The bio-implantable actuator of claim 9, further comprising a tension adjuster that is able to adjust tension of the elastic member.

11. The bio-implantable actuator of claim 10, wherein the tube includes:
a coupling hole for fixing to a femur; and
an adjustment hole connecting the hollow portion of the tube and an outer portion of the tube.

12. The bio-implantable actuator of claim 11, wherein the tension adjuster includes:
a tension adjustment bolt disposed in the adjustment hole of the tube;
a screw disposed inside the tube; and
a second connecting member that is formed of a soft material and connects the tension adjustment bolt and the screw.

13. The bio-implantable actuator of claim 12, wherein the linear movement section further includes a third connecting member that is movably disposed at the screw and is coupled to the flexible rod.

14. The bio-implantable actuator of claim 13, wherein the tension adjuster further includes:
an adjustment nut adjusting a compressed length of the elastic member; and
a pin coupling the adjustment nut to the third connecting member, and
the third connecting member includes a pin hole to which the pin is coupled.

15. An artificial joint comprising:
the bio-implantable actuator of claim 1;
a femur connector fixed to a lower end of a femur;
a tibia tray fixed to an upper end of a tibia; and
a bearing that is coupled to an upper end of the tibia tray and has a curved surface formed thereon,
wherein the bio-implantable actuator is coupled to the tibia tray, and
the femur connector is disposed to be slidable along the curved surface of the bearing.

16. The artificial joint of claim 15, wherein the bearing has a through-hole through which the tube and the flexible rod pass.

17. The artificial joint of claim 16, wherein a width of the through-hole is larger than a diameter of the tube.

18. The artificial joint of claim 17, wherein the tibia tray includes:
a first coupling groove coupled to the through-hole of the bearing; and
a second coupling groove coupled to the flexible rod.

19. The artificial joint of claim 18, wherein the second coupling groove has a narrowing width on at least a portion thereof in a direction toward a bottom surface and guides a bending direction of the flexible rod.

20. The artificial joint of claim 19, wherein the flexible rod is bent along the second coupling groove upon receiving a pressure.
